# EUROPEAN PATENT SPECIFICATION

(11) **EP 3 363 899 B1**
(45) Date of publication and mention of the grant of the patent: **17.03.2021**
(21) Application number: 16862499.7
(22) Date of filing: 04.11.2016
(51) Int. Cl.: C12N 15/10, C12Q 1/68, G01N 33/68, C40B 40/08, C40B 40/06, C12Q 1/6848

(54) **MOLECULAR CLONE EXTRACTING AND VERIFYING METHOD**
VERFAHREN ZUR EXTRAKTION UND VERIFIZIERUNG MOLEKULARER KLONE
PROCÉDÉS D'EXTRACTION ET DE VÉRIFICATION DE CLONES MOLÉCULAIRES

(30) Priority: 04.11.2015 KR 20150154483; 19.09.2016 KR 20160119420
(43) Date of publication of application: 22.08.2018
(73) Proprietor: Celemics, Inc., Geumcheon-gu, Seoul 08506 (KR); Seoul National University R&DB Foundation, Seoul 08826 (KR)
(72) Inventor: RYU, Tae Hoon, Gimpo-si Gyeonggi-do 10114 (KR); KIM, Hyo Ki, Seoul 08734 (KR); HAN, Hyo Jun, Seoul 08080 (KR); KWON, Sung Hoon, Seoul 06291 (KR)
(74) Representative: Prüfer & Partner mbB Patentanwälte · Rechtsanwälte
(86) International application number: PCT/KR2016/012699
(87) International publication number: WO 2017/078484

(56) References cited:
- EP-A1- 2 919 008
- WO-A1-2015/103225
- WO-A2-2007/133710
- WO-A2-2013/163263
- KR-A- 20140 075 611
- KR-A- 20140 111 224
- KR-A- 20150 090 352
- US-A1- 2015 080 266
- HOWON LEE ET AL: "A high-throughput optomechanical retrieval method for sequence-verified clonal DNA from the NGS platform", NATURE COMMUNICATIONS, vol. 6, no. 1, 2 February 2015 (2015-02-02), XP055592991, DOI: 10.1038/ncomms7073
- SHOKRALLA ET AL.: 'Next-geueration DNA Barcoding: Using Next-generation Sequencing to Enhance and Accelerate DNA Barcode Capture from Single Specimens' MOLECULAR ECOLOGY RESOURCES vol. 14, 2014, pages 892 - 901, XP055365213

## Description

### Technical Field

The present disclosure relates to a method for extracting and characterizing molecular clones.

### Background Art

Antibodies are glycoproteins that are produced from antigens (for example, viruses and bacteria) stimulating *in vivo* immune responses and specifically bind to antigens to induce extracellular stimuli. Antibodies bind to specific proteins with high selectivity. For this reason, antibodies are of great utility in the fields of biology, biotechnology and medicine, including from basic studies associated with labeling/observation of target proteins to medical applications associated with activity regulation of abnormal proteins in patients' bodies. Particularly, the utility of antibodies as platforms for next-generation new drugs, such as antibody drug conjugates (ADCs) and chimeric antigen receptor-T cell (CART) therapeutics, has increased continuously in the recent development of new drugs.

Generally, the usefulness of antibodies is determined by their ability to bind to and specificity for target proteins. Herein, specificity refers to the property of antibodies that bind to target proteins but not others. Such a binding property results from the tertiary structural features of antibodies. The tertiary structure of antibodies varies depending on changes in the amino acid sequence of the antibodies. The modification of the tertiary structure of antibodies by changing deoxyribonucleic acid (DNA) sequences corresponding to specific amino acid residues of the antibodies and the characterization of the structure-modified antibodies are widely used methodologies in the field of antibody engineering. These methodologies require techniques for sequencing antibodies and techniques for selecting DNA molecules having specific sequences, which occupy a very important position in the entire procedure for antibody development.

Generally, the sequencing of antibodies and the selection of DNAs of desired antibodies are accomplished through cloning and Sanger sequencing. However, they are very cost- and time-consuming, making it difficult to develop antibodies.

EP 2 919 008 for example discloses a method of isolating biochemical molecules on a microarray substrate, wherein this method comprises steps S1 to S4. In step S1, a microarray substrate in which clusters of biochemical molecules are classified and fixed by individual spot units is provided; in step S2, location information of individual spots in which desired biochemical molecules locate is obtained, step S3 comprises locating an extraction tool for applying energy according to the location information, and step S4 comprises isolating the desired cluster from the microarray substrate by applying energy using the extraction tool. It is further disclosed that amplified DNA was subjected to Sanger sequencing.

In efforts to solve such problems, methods based on next generation sequencing (NGS) have been proposed. However, these methods can analyze only short sequences, which limits their practical use. The present inventors have conducted research to overcome the above-mentioned technical limitations and to sequence antibodies at low cost. Thus, the present inventors propose a method for rapidly extracting molecular clones and sequencing the molecular clones based on DNA barcoding and next generation sequencing (NGS) before use of the molecular clones.

### Detailed Description of the Invention

### Means for Solving the Problems

The present disclosure provides a method and system for characterizing individually separated molecular clones. Sanger sequencing has been traditionally used as a tool to sequence molecular clones. In recent years, next generation sequencing has been used in biology, biotechnology and related fields and in various applications across all industries. However, the effectiveness of next generation sequencing for molecular clones is not satisfactory from an economic viewpoint.

According to one aspect of the present invention, there is provided a method for extracting and characterizing molecular clones, including: providing a substrate on which molecular clones are formed; applying energy to desired ones of the molecular clones in a non-contact mode to extract the desired molecular clones from the substrate; chemically linking DNA barcodes to the sequences of the extracted molecular clones; and determining the DNA barcode-linked sequences by parallel sequencing.

The non-contact extraction of the desired molecular clones enables rapid extraction of the molecular clones and does not require the use of consumables, so that the extraction cost can be minimized. In addition, the attachment of the DNA barcodes with known sequences to the molecular clones provides information on the locations of the corresponding molecular clones, enabling sequencing of the molecular clones by next generation sequencing. According to the method of the present disclosure, next generation sequencing may be used instead of Sanger sequencing so that molecular clones can be rapidly analyzed on a large scale at greatly reduced cost. Therefore, the method of the present disclosure enables the synthesis of DNA nucleotides at low cost and is thus expected to encourage technological developments in the field of gene synthesis.

### BRIEF DESCRIPTION OF THE DRAWINGS

Fig. 1 is a flow chart illustrating a method for extracting and characterizing molecular clones according to one embodiment of the present disclosure.
Fig. 2 is a schematic process flow diagram illustrating a method for the extraction and analysis of molecular clones according to one embodiment of the present disclosure.
Fig. 3 shows an image of emPCR products according to one embodiment of the present disclosure.
Fig. 4 shows shapes and arrangements of molecular clones according to one embodiment of the present disclosure.
Figs. 5 and 6 are schematic diagrams of a system for extracting molecular clones according to one embodiment of the present disclosure.
Fig. 7 illustrate schematic diagrams of various processes for extracting molecular clones according to exemplary embodiments of the present disclosure.
Fig. 8 is a schematic diagram illustrating a process for attaching DNA barcodes according to one embodiment of the present disclosure.
Fig. 9 shows the sequences of exemplary DNA barcodes used in experiments.
Fig. 10 shows amplification products of molecular clones attached with DNA barcodes according to exemplary embodiments of the present disclosure.
Fig. 11 shows some parallel sequencing results according to exemplary embodiments of the present disclosure.
Fig. 12 shows the sequences of retrieved molecular clones according to exemplary embodiments of the present disclosure.

### Mode for Carrying out the Invention

The present invention will be described in detail with reference to various embodiments. A general method for extracting and characterizing molecular clones includes 1) obtaining molecular clones from a microbe such as *E. coli,* 2) manually extracting the molecular clones, 3) increasing the amount of DNA in the molecular clones by culture or DNA amplification (such as polymerase chain reaction (PCR)), and 4) analyzing the amplified DNA by Sanger sequencing. In contrast, a method of the present disclosure enables rapid acquisition of information on the sequences of molecular clones and retrieval of the molecular clones based on a convergence technology of non-contact extraction, DNA barcoding, and next generation sequencing of molecular clones at low cost compared to conventional methods. These advantages are attributed to the features of non-contact extraction and next generation sequencing of molecular clones. The method of the present disclosure utilizing the characterized molecular clones enables gene synthesis at at least 10-fold lower cost than conventional methods.

Embodiments of the present invention will now be described in detail with reference to the accompanying drawings. These embodiments are provided so that this disclosure will be thorough and complete, and will fully convey the scope of the invention to those skilled in the art. Accordingly, the present invention may be embodied in many different forms and should not be construed as limited to the exemplary embodiments set forth herein. In the drawings, the dimensions, such as widths, lengths and thicknesses, of elements may be exaggerated for clarity. The drawings are explained from an observer's point of view. It will be understood that when an element is referred to as being "on" another element, it can be directly on the other element, or one or more intervening elements may also be present therebetween.

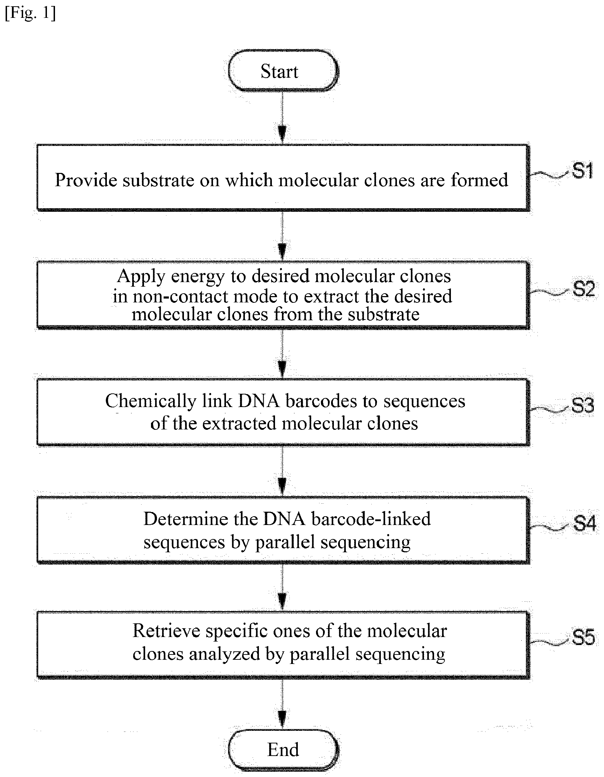

One aspect of the present invention provides a method for extracting and characterizing molecular clones as defined in claim 1. Fig. 1 is a flow chart illustrating a method for extracting and characterizing molecular clones according to one embodiment of the present disclosure.

In step S1, a substrate on which molecular clones are formed is provided. The molecular clones possess the same nucleic acid sequence information on a clone basis. The molecular clones can be obtained by amplifying certain nucleic acid molecules in a space physically separated from other nucleic acid molecules. The nucleic acid may include deoxyribonucleic acid (DNA), ribonucleic acid (RNA), peptide nucleic acid (PNA), locked nucleic acid (LNA), glycol nucleic acid (GNA), threose nucleic acid (TNA), xeno nucleic acid (XNA), synthetic nucleic acid, modified nucleic acid, and combinations thereof. The nucleic acid is preferably DNA due to its ease of *in vitro* or intracellular amplification.

The nucleic acid molecules may be amplified by any suitable method using polymerase, such as polymerase chain reaction (PCR), *in vitro* transcription, reverse transcription, linear amplification, multiple displacement amplification (MDA), rolling circle amplification (RCA), emulsion PCR, emulsion PCR using beads, bridge PCR, intracellular infusion, intracellular cloning or a combination thereof. Cells that can divide may be used without particular limitation in the method of the present disclosure Examples of such cells include microbial cells, such as *E. coli* or yeast cells, and mammalian cells. The use of rapidly dividing microbial cells is advantageous for the amplification of nucleic acid molecules except for special cases. Emulsion PCR using beads or intracellular cloning is preferred because the use of beads or cells facilitates subsequent extraction of molecular clones.

The special cases include *in vivo* expression of proteins, such as antibodies, expression of specific genes, insufficient cloning in specific cells. transfection of bacteriophage or virus into host cells, and the presence of enzymes (for example, restriction enzymes or transposable elements) affecting specific sequences in cells.

The substrate is a solid-state substrate such as glass or silicon. In a broad sense, the substrate is intended to include gel-like substrates such as polyacrylamide and agarose.

Other substances such as molecules or cells can be introduced onto the substrate by bonding or adsorption via the molecular clones formed on the substrate. Since the molecular clones may have different DNA sequences or corresponding RNA sequences or protein structures thereof, they can be distinguished from other libraries based on chemical features resulting from the structural differences. Based on this principle, when the individual molecular clones are separated, the other libraries can be effectively separated. The other libraries refer to libraries of biochemical molecules such as nucleic acids, modified nucleic acids, proteins, peptides, and liposomes, libraries of chemical molecules such as small molecules, libraries of viruses, and libraries of cells such as *E*. *coli,* yeast, leukocytes, cancer cells, and stems cells. The other libraries may include libraries having a volume of 1 aL to 1 µL. Preferred libraries that can be combined with the molecular clones formed on the substrate are nucleic acids, nucleic acid binding proteins, cells surface displayed with nucleic acid binding proteins, and proteins binding to specific surface proteins. The combination has an advantage in that the other libraries can be easily sorted based on their known constructional and biochemical properties. Examples of the nucleic acid binding proteins include zinc fingers and transcription activator-like effectors (TALEs). More broadly, the nucleic acid binding proteins may be RecA, Cas9, and analogous proteins which bind to DNA or RNA via DNA or RNA, and transposomes.

The substrate may be selected from the group consisting of a substrate replicated from a template, a substrate including a sacrificial layer therein, a substrate surface coated with a sacrificial layer, and a substrate undergoing a phase transition under an electromagnetic field. The efficiency of these substrates can be enhanced when energy is applied to the substrates in the extraction step. It is preferable to place a sacrificial layer on the substrate because the sacrificial layer absorbs energy to increase the extraction efficiency and reduces the amount of energy applied to biochemical molecules to minimize damage to the biochemical molecules. The sacrificial layer may be a glass or silicon layer that lowers transmittance or increases absorbance to increase the absorption of energy. The position of the sacrificial layer is not limited. For example, the sacrificial layer may be coated with on the surface of a solid such as glass or silicon. Alternatively, the sacrificial layer may be present inside a solid such as glass or silicon. The substrate undergoing a phase transition under an electromagnetic field means a solid substrate that is temporarily or permanently liquefied, vaporized or plasmarized. The substrate replicated from a template refers to a substrate that is manufactured through large-area transfer or replication [Haohao Lin et al., Replication of a DNA microarray, JACS 127, 11210-11211 (2005), Haohao Lin et al., Replication of a DNA microarray from zip code masters, JACS 128, 3268-3272 (2006)] or a substrate that is manufactured by stamping a colony plate.

The molecular clones are usually located in a random arrangement on the substrate. This random arrangement is typically found when a microbe is spread on an agar plate during general cloning. This arrangement can also be observed after a predetermined amount of a bead solution is applied to the substrate. However, regularly arranged molecular clones can be obtained when a microwell is used as the substrate. A regular arrangement of molecular clones is advantageous compared to a random arrangement in that the extraction and DNA barcoding of molecular clones can be easily performed. The molecular clones are spaced at least 1 µm from the adjacent molecular clones. This arrangement facilitates the extraction of the molecular clones. The molecular clones have a size that can be observed with naked eyes or under a microscope. Generally, the molecular clones have a diameter of 100 µm or less (usually 1 µm or more) for their ease of extraction. For example, the molecular clones may take the form of microbial colonies with a diameter of 100 µm or less.

The substrate may include a microwell structure as an array of regularly arranged wells, each of which has a volume of 1 µL or less.

The term "regular arrangement" means an arrangement in which isosceles triangles, right-angled triangles, equilateral triangles, rhombuses, parallelograms, rectangles, squares, regular pentagons, regular hexagons, or regular octagons to regular 30-gons, preferably right-angled triangles, rectangles, squares or regular hexagons, more preferably rectangles, squares or regular hexagons are drawn when the central points of spots are connected. The regular arrangement is not limited and may include any design whose regularity can be found. The intervals between the spots may be from 1 nm to 5 mm, preferably from 100 nm to 1 mm, more preferably from 300 nm to 1 mm, even more preferably from 1 µm to 500 µm, particularly 5 µm to 100 µm.

In step S2, energy is applied to desired ones of the molecular clones in a non-contact mode to extract the desired molecular clones from the substrate. The application of energy in a non-contact mode may be performed by at least one mode selected from the group consisting of ultrasonic wave application, pneumatic pressure, and laser application. The non-contact mode is advantageous in terms of cost because no cross-contamination is caused and the use of consumables is minimized.

Preferably, the application of energy in a non-contact mode is based on pulse laser ablation by an incident pulse laser or radiation pressure ejection. In this case, some or all of the molecular clones are separated from the substrate. The direction of propagation of the laser wavelength is substantially the same as the moving direction of the molecular clones, making it easier to retrieve the separated molecular clones.

The pulse laser may have a wavelength of 10 to 10,000 nm, preferably 20 to 5,000 nm, more preferably 100 to 2,000 nm. An electromagnetic field in the wavelength range defined above, including the visible or infrared range, has no significant influence on optical elements and can transfer sufficient energy to the substrate or the molecular clones. Most commercial pulse lasers operate in the above range and are thus easy to realize the system. Also when the substrate uses a sacrificial layer, the method of the present disclosure can be carried out without any substantial change of the system.

The pulse laser may have a pulse duration in the range of 1 as to 1 ms, preferably 1 fs to 100 ns. When pulse laser ablation is performed by the pulse laser having a pulse duration in the range defined above, the propagation paths of the separated substrate and molecular clones are made more constant, making it easy to retrieve the molecular clones. The pulse laser has an output of 10 to 1 kJ/cm² per pulse, preferably 100 to 300 J/cm² per pulse. When pulse laser ablation is performed by the pulse laser having a pulse duration and a pulse output in the ranges defined above, nucleic acid molecules of the molecular clones are less damaged, bringing about high efficiency during subsequent processing of the separated nucleic acids.

The separation of the desired molecular clones from the substrate includes transferring the desired molecular clones to a reservoir. The transfer to the reservoir is necessary to store the separated molecular clones and use the molecular clones upon reaction with other reactants. The reservoir may include a container designed to cause or observe physical or chemical reactions. The reservoir may include a container designed to store the biochemical molecules. The reservoir has a volume of 1 aL to 1 L, preferably 1 fL to 10 mL, more preferably 1 pL to 500 µL, which corresponds to a reaction volume in which post-processing of the molecular clones and the nucleic acids separated through biochemical reactions can be most easily performed. The reservoir may be an array of microwells, each of which has a volume of 1 pL to 1 µL [David K. Wood *et al.,* Single cell trapping and DNA damage analysis using microwell arrays, PNAS (2010)]. This array structure can reduce the reaction volume for chemical reactions of the separated molecular clones and the nucleic acids to minimize the waste of reagents. In addition, improved reaction rates and efficiencies in some of the reactions can be expected.

The desired molecular clones are separated from the substrate by transferring some or all of the molecular clones to the reservoir. The separation of portions of the molecular clones enables the use of the corresponding molecular clones one or more times. Thus, when the same molecular clones are necessary later, the extracted molecular clones can be rapidly retrieved from the corresponding locations of the reservoir that have been previously identified. The desired molecular clones can be separately stored in one reservoir, and as a result, chemical reactions between the molecular clones and the nucleic acids can be induced.

The tool for extraction of the molecular clones may be combined or linked with a system for image observation and storage. The tool may be combined with an arithmetic unit that can drive an object recognition algorithm for analyzing the images to determine the shape and location of the molecular clones. These elements are essential for automatic extraction of the molecular clones.

The extraction tool may be combined with a device for moving the tool. The device may be electrically driven. The device is preferably manipulated with a precision of 1 mm or less, more preferably 100 µm or less, even more preferably 5 µm or less. Since the intervals between spots in most microarrays are from 5 µm to 100 µm, the electrically driven device that is manipulated with a precision of 5 µm or less enables accurate separation of the desired molecular clones in most microarrays.

The extracted molecular clones may be in the form of microbes or cells. In this case, the method of the present disclosure may further include filling a culture medium in the reservoir and culturing the molecular clones to amplify the amount of nucleic acids in the molecular clones.

In step S3, DNA barcodes are chemically linked to the sequences of the extracted molecular clones. For example, DNA barcodes may be chemically linked to both ends of the sequence of each molecular clone. Preferably, the chemical linking is performed using an enzyme. Examples of enzymes suitable for the chemical linking include polymerases, ligases, and transposons (e.g., Tn5).

The enzyme is preferably a polymerase in terms of processing and economic efficiency. Generally, the content of the nucleic acids in the extracted molecular clones is at the fmole level or less, making it difficult to use the molecular clones without amplification. Since the nucleic acids need to be amplified for subsequent processing, the use of a polymerase is preferred due to the ability of the enzyme to simultaneously link the barcodes to the nucleic acids and amplify the nucleic acids. When the nucleic acids, particularly DNAs or RNAs, present in the molecular clones are circular rather than linear in shape, it is impossible to use a ligase as the enzyme. In addition, polymerases are generally produced at low cost compared to other enzymes because of their ease of purification. Suitable barcoding methods using polymerases include polymerase chain reaction (PCR), rolling circle amplification (RCA), multiple displacement amplification (MDA), and assembly PCR (also called PCA or overlap extension PCR).

Preferably, the DNA barcodes have sequences consisting of 5 or more bases, preferably 7 or more bases. Parallel DNA synthesis using microarrays enables the synthesis of DNAs, for example, ∼10,000 different DNAs, and also increases the complexity of antibody libraries to at least 10,000. Thus, there is a need to secure a sufficient number of different DNA barcodes that are to be attached to 10,000 or more molecular clones. Since barcodes can be attached to both ends of each molecular clone, at least 100 barcodes are required when the number of combinations of terminal barcodes is taken into account. The sequences consist of at least 4 bases but it is advantageous to construct the barcodes such that the sequences consist of 5 or more bases or 7 or more bases in consideration of sequencing errors, ease of barcode attachment, and potential problems caused by the secondary structure of the barcodes.

Preferably, homopolymers of 3 or more bases are excluded from the barcodes. The proportion of GC in the barcodes is about 30-70%. This proportion is helpful in conducting molecular biology experiments for barcoding. A certain barcode is designed such that it does not share 5 or more bases with other barcodes. This design can overcome difficulties in barcode analysis caused by DNA synthesis errors.

It is recommended to use two or more sets of barcodes in order to increase the number of cases that can be represented by the DNA barcodes. For example, in the case where set A consisting of 96 barcodes and set B consisting of 96 barcodes are attached to the ends of DNAs originating from the molecular clones, a total of 96^{∗}96 = 9216 codes can be obtained from the 192 barcodes. When the combinatorial barcoding using two or more sets of DNA barcodes is used, there is no need to attach the DNA barcodes from the different sets to the ends of the nucleic acid molecules in a one-to-one relationship. The barcodes from the different barcode sets may be connected to one end of each nucleic acid molecule in series.

Multiplex PCR and a polymerase may be used to link the barcodes. Multiplex PCR refers to a PCR technique that uses two or more pairs of primers or amplifies two or more different regions. In many cases, the length of DNA encoding antibodies or amino acids of scFv is larger than that of DNA readable at one time by a general next generation sequencing technique. Accordingly, portions of the constituent DNA of an antibody need to be amplified separately to determine the full-length sequence of the antibody. Multiplex PCR is an optimal technique for this purpose.

In step S4, the DNA barcode-linked sequences are determined by parallel sequencing. For example, sequencing by synthesis, sequencing by ligation, and nanopore sequencing with HiSeq and MiSeq from Illumina, Ion Torrent from Life Technology, and 454 from Roche may be used for parallel sequencing. The extracted molecular clones are placed in different reservoirs in step S2 and are coupled to the DNA tags in step S3. Accordingly, in step S4, a determination can be made as to which sequences are located in the reservoirs based on the obtained information.

In step S5, specific ones of the molecular clones analyzed by parallel sequencing are retrieved. There are various criteria for the selection of specific molecular clones. Generally, specific molecular clones are selected for protein expression, RNA expression, and insertion into vectors. Molecular clones of particular interest (or economically useful molecular clone) are preferentially selected. The retrieved molecular clones may be in the form of barcoded linear dsDNAs. In the case where the molecular clones are in the form of microbes or cells, the molecular clones may be retrieved by extracting the molecular clones and acquiring microbes or cells remaining in the culture solution. The information obtained by the parallel sequencing contains information on the specific sequences and information on the barcodes corresponding to the locations of the reservoirs in which the molecular clones having the corresponding sequences are present. As a result, the specific molecular clones can be retrieved by determining the locations of the reservoirs through the barcode information.

The molecular clones may be retrieved by removing the DNA barcodes from the barcoded molecular clones. In this case, PCR using a universal primer is generally performed one more time on the DNA barcode-attached molecular clones to remove the barcoded regions. When it is intended to use the retrieved molecular clones for special purposes such as gene synthesis, assembly products of the corresponding molecular clones in which the DNA barcodes remain may create unwanted products other than desired sequences. Accordingly, it is preferred to remove the DNA barcodes when the molecular clones are retrieved for use in specific applications.

In addition, the method of the present disclosure may further include expressing and producing RNAs or proteins using the DNAs of the retrieved molecular clones.

An *in vitro* transcription system may be used for RNA expression and an in vitro transcription/translation system may be used for protein expression.

The protein expression and production may be accomplished using cells or bacteriophages. Examples of suitable cells include, but are not limited to, *E. coli,* yeast, and CHO cells. Bacteriophages having the function of expressing proteins on their surface are mainly used. M13 is a representative species of bacteriophages. Other species of bacteriophages may also be used. A variety of protein expression vectors may be used for protein expression. When bacteriophages are used, pComb3X may be used as a vector. This vector is advantageous in expressing protein on the surface of bacteriophages and propagating bacteriophages in *E. coli.*

The method of the present disclosure may further include performing various analyses using the proteins. In step S5, it is general to selectively retrieve molecular clones of potential industrial value. However, the selectively retrieved molecular clones need to be actually verified for their usefulness. Accordingly, it is necessary to express proteins using the molecular clones and analyze various characteristics of the proteins. The structural features of the proteins and the binding strengths of the proteins to other substances (for example, viral proteins) due to the structural features of the proteins are generally of importance. X-ray crystallography can be used to investigate the structural features of the proteins. The structure of the proteins can also be analyzed by simulations.

The binding strengths of the expressed proteins to other substances are preferably confirmed by an enzyme-linked immunosorbent assay (ELISA) technique. The binding strengths can also be confirmed by attaching fluorescent molecules to antigen proteins without using enzymes and directly observing the fluorescence. The ELISA technique may be microwell ELISA using microfluidics and microelectromechanical systems (MEMSs) but is not limited thereto. Microwell ELISA has the advantage of cost saving. A suitable mode selected from direct, sandwich, and competitive modes may be used to select the specific molecular clones.

According to one embodiment, the method of the present disclosure may further include selectively retrieving only desired ones of the characterized molecular clones and using the desired molecular clones for target sequencing, the construction of capture probes for exome or whole exome sequencing (WES) or gene synthesis. For example, specific sequences may be selectively retrieved by subjecting the selectively retrieved molecular clones to amplification, transcription, and hybridization with gDNA. By analyzing the retrieved specific sequences, target sequencing or exome sequencing can be effectively performed. Particularly, since individual capture probes are separated from each other, the capture efficiency can be deliberately controlled by varying the concentrations of the capture probes.

The separated nucleic acids can be assembled into genes by at least one method selected from amplification, ligation, assembly PCR, isothermal assembly [Daniel G Gibson et al., Enzymatic assembly of DNA molecules up to several hundred kilobases, Nature Methods 6, 343-345 (2010)], and pairwise assembly [William J. Blake et al., Pairwise selection assembly for sequence-independent construction of long-length DNA, Nucleic Acids Research 38, 2594-2602 (2010)].

According to one embodiment, the retrieved molecular clones may be cloned into pComb3X, transformed in *E. coli,* and expressed in bacteriophages to determine their binding strengths to antigens.

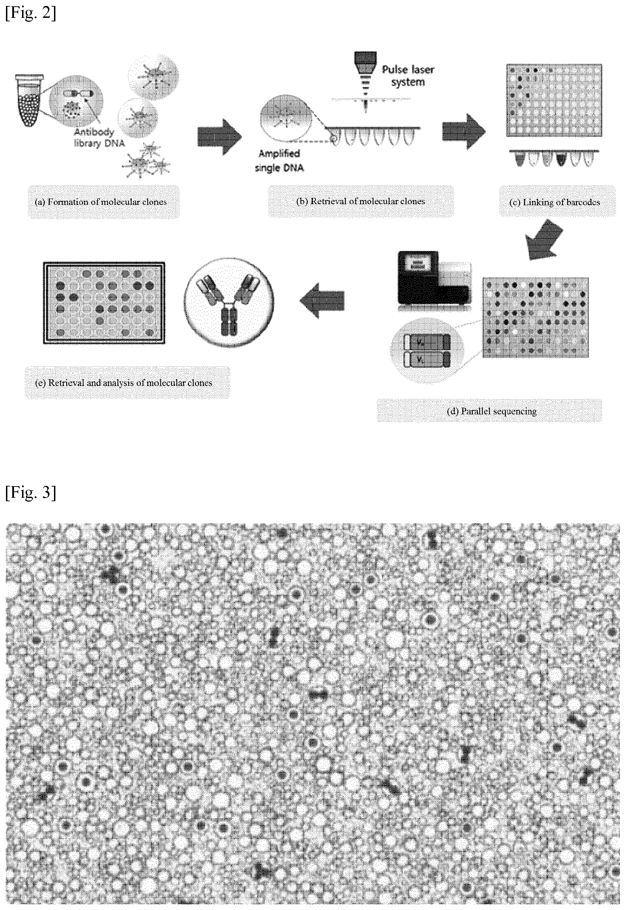

Fig. 2 is a schematic process flow diagram illustrating a method for the extraction and sequencing of molecular clones according to one embodiment of the present disclosure. (a) of Fig. 2 shows a process for forming molecular clones by emPCR. This process may be carried out by cloning using cells such as microbial cells. DNAs are generally used as nucleic acids to form the molecular clones. The DNAs may be synthesized in microarrays or may be antibody library DNAs. (b) of Fig. 2 shows a process for extracting the molecular clones. The molecular clones formed in the process shown in (a) of Fig. 2 are immobilized on a substrate, separated from the substrate in a non-contact mode using a pulse laser system, and transferred to an underlying reservoir. (c) of Fig. 2 shows a process for attaching different DNA barcodes to the molecular clones extracted from the substrate and transferred to the reservoir. Generally, different barcodes capable of specifically labeling wells are attached to the molecular clones. (d) of Fig. 2 shows a process for acquiring information on the sequences through parallel sequencing and information on the barcodes corresponding to the sequences. For antibodies, information on VH region and information on VL region may be separately acquired and the sequences of VH and VL may be reconstructed through a bioinformatics approach via barcodes in a subsequent process. (e) of Fig. 2 shows a process for retrieving the molecular clones and characterizing the antibodies through ELISA.

Fig. 3 shows an image of emPCR products according to one embodiment of the present disclosure. PCR is performed on 1-3 beads trapped in an emulsion. Each bead has 0.1 DNA molecules on average. Accordingly, most of the beads constitute single molecular clones.

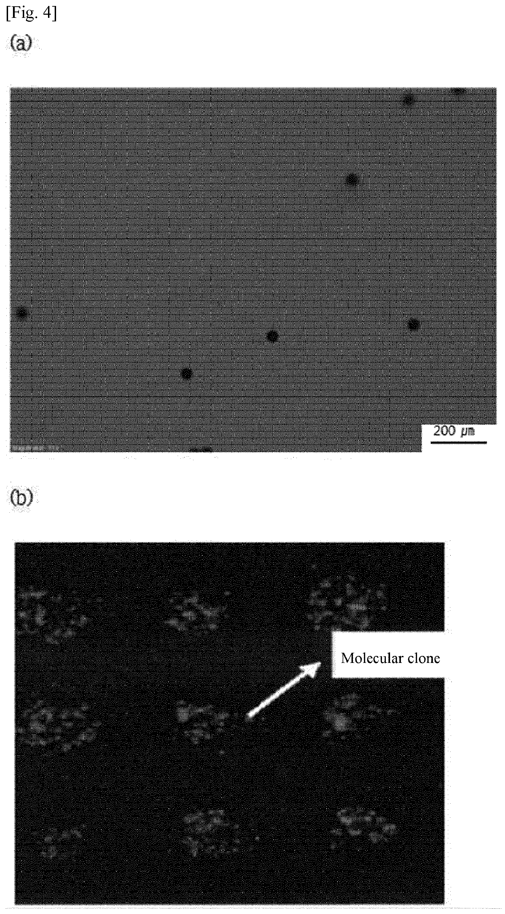

Fig. 4 shows shapes and arrangements of molecular clones according to one embodiment of the present disclosure. (a) of Fig. 4 shows a random arrangement of molecular clones found after a predetermined amount of a bead solution was applied to a substrate. The molecular clones were formed by emulsion PCR (emPCR) using beads. (b) of Fig. 4 shows molecular clones formed by culture after *E*. *coli* cells were plated in regularly arranged microwells one by one. In both (a) and (b) of Fig. 4, the molecular clones are easy to separate in a non-contact mode due to their large size (∼100 µm). The spaces where the *E. coli* cells are cultured may not be regularly arranged. In this case, microcolonies are simple to form but the position of *E. coli* needs to be confirmed through image processing.

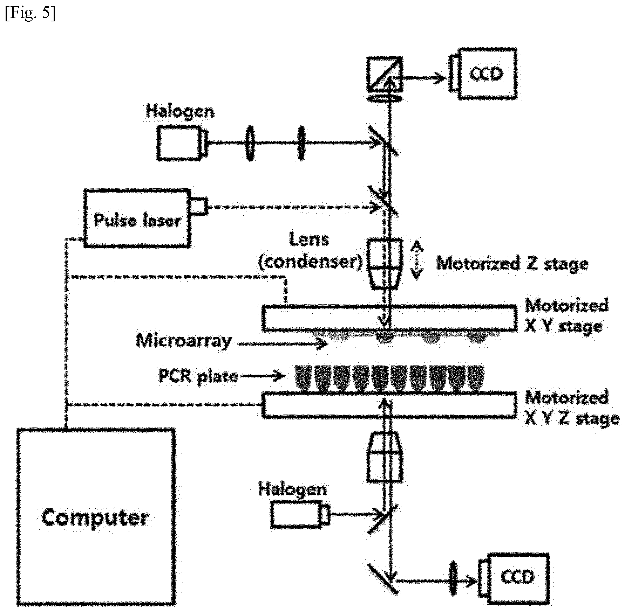

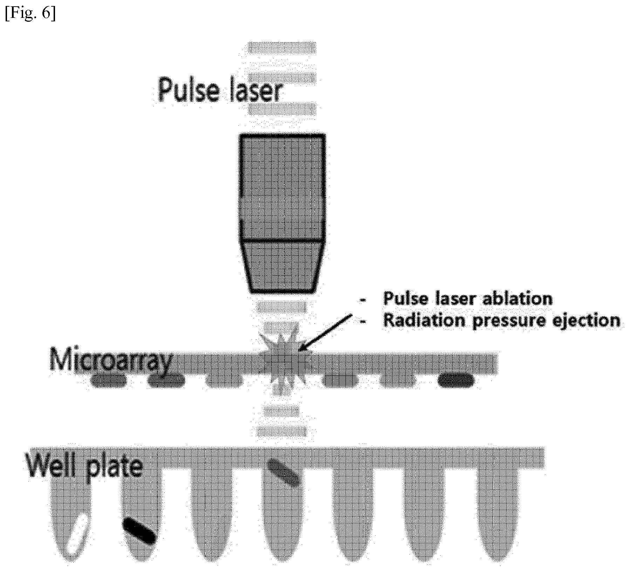

Figs. 5 and 6 are schematic diagrams of a system for extracting molecular clones according to one embodiment of the present disclosure. The system includes a stage on which a substrate attached with molecular clones is mounted, an extraction unit for applying energy to desired ones of the molecular clones in a non-contact mode to separate the desired molecular clones from the substrate, and a control unit for locating the substrate such that the specific areas of the substrate correspond to the extraction unit, to separate the desired molecular clones.

According to one embodiment, the extraction unit may include a pulse laser source and a condenser. The condenser is preferably an optical lens. The optical lens can be used to focus pulse laser energy and to observe the molecular clones and the substrate. The pulse laser can be accurately irradiated onto the locations of the molecular clones to be separated on the substrate at desired points of time, which is usually performed by controlling the pulse laser using the control unit such as a computer. The optical lens may have a magnification ranging from 2× to 100×, preferably from 10× to 40×. Within this range, energy suitable for separating the molecular clones can be transmitted to the substrate and the lens can be prevented from coming into contact with the microarray substrate or detracting from the focal distance.

According to one embodiment, at least one of the stage, the extraction unit, and the control unit of the extraction system may be manipulated with a precision of 1 mm, preferably 100 µm, more preferably 1 µm or less. The stage, the extraction unit, and the control unit, which can be manipulated with a precision of 1 µm or less, enable accurate separation of desired molecular clones on most substrates because the intervals between most molecular clones are 1 µm or above.

According to one embodiment, the system may further include an imaging unit for observing the substrate so as to separate the desired molecular clones. The imaging unit may consist of one or more elements selected from an optical lens, a light source, and an image sensor. The optical lens may be included in the extraction unit. If needed, a separate optical lens may be further used.

The light source has a wavelength in the range of 10 nm to 10,000 nm, preferably 50 nm to 2,000 nm, more preferably 100 nm to 1,500 nm. Within this range, the substrate can be most easily observed or monitored using fluorescence or visible light. The light source may be, for example, a halogen lamp.

The image sensor is usually a charge-coupled device (CCD) but is not limited thereto. The image sensor may be used in step S2 to acquire the locations of individual spots in which the desired ones of the molecular clones on the substrate are located and to confirm whether the spots are present in the locations. The imaging unit may be used in step S2 to confirm whether an extraction tool for applying energy to separate the desired molecular clone depending on the location information is accurately located. The imaging unit may be used in step S2 to confirm whether the desired molecular clones separated from the substrate are present in the reservoir.

According to one embodiment, the system may further include another stage on which the reservoir for retrieving the separated desired molecular clones is mounted. The stage may be manipulated with a precision of 1 mm or less, preferably 100 µm or less, more preferably 1 µm or less. The additional stage on which the reservoir is mounted can facilitate the utilization of the separated biochemical molecules. The stage having a precision of 1 µm or less enables the separation of the biochemical molecules into the reservoir having an array of microwells, each of which has a volume of 1 pL to 1 µL.

The extraction system illustrated in Fig. 5 is broadly divided into an upper system and a lower system. The upper system is controlled by a computer and consists of an upper stage under which the substrate is attached (Motorized XY stage), an energy application device, and an upper imaging device.

According to one embodiment, a pulse laser beam is focused and enters the substrate through the condenser, and the molecular clones on the substrate are extracted and pushed to an underlying PCR plate as a reservoir through expansion pressure by pulse laser ablation or radiation pressure. The condenser may be an optical lens. For example, the optical lens may have a magnification ranging from 2× to 100×. The lower system includes a lower stage moveable in the Z-axis direction (Motorized XYZ stage), a PCR tube rack or PCR plate as a reservoir attached on the lower stage, and a lower imaging device. In the lower imaging device, a reservoir through which image can pass may be used for optical observation when the molecular clones separated from the substrate are collected or to determine a physical reference location of the well. For example, the reservoir includes a variety of accommodation tools. The reservoir may be a flat bottom reservoir or a flat bottom PCR plate made of a transparent plastic material. The flat bottom of the reservoir does not substantially affect the path of light from the light source of the lower imaging device, enabling easy imaging.

Fig. 6 is a schematic diagram showing the principle of extraction of the molecular clones according to one embodiment of the present disclosure. The use of the pulse laser enables the extraction of the molecular clones through a variety of mechanisms of action. Representative examples of such mechanisms include pulse laser ablation and radiation pressure ejection.

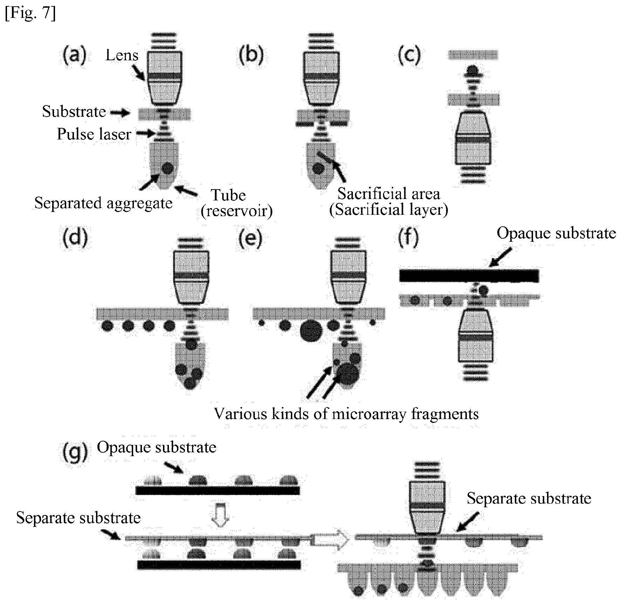

Fig. 7 illustrate schematic diagrams of various processes for extracting molecular clones according to exemplary embodiments of the present disclosure. (a) of Fig. 7 shows a process for directly irradiating a laser onto molecular clones (aggregates) to extract the molecular clones. (b) of Fig. 7 shows a process for extracting molecular clones by attaching the molecular clones to a substrate coated with a sacrificial layer and irradiating a laser onto the sacrificial layer. According to this process, kinetic energy can be transferred through the sacrificial layer, allowing for extraction of the molecular clones that are difficult to directly extract with a laser. (c) of Fig. 7 shows a process for extracting molecular clones in a state in which a lens, a substrate, and molecular clones are inverted. This process is useful when it is desired to control the movement of extracted molecular clones in microfluidic channels. (d) of Fig. 7 shows a process in which various kinds of molecular clones are extracted and collected the extracted molecular clones in one reservoir. (e) of Fig. 7 shows a process in which molecular clones with different sizes (various kinds of microarray fragments) are extracted and collected in one or several reservoirs. (f) of Fig. 7 shows a process for extracting molecular clones using light reflected from the molecular clones present on an opaque substrate when irradiated with a laser. In this process, it is necessary to change the direction of the laser irradiation and the locations of the molecular clones on the substrate, as shown in (f) of Fig. 7, because light is not transmitted through the opaque substrate. (g) of Fig. 7 shows a process for extracting replicated molecular clones. According to this process, a substrate formed with molecular clones is previously replicated when it is intended to retrieve specific molecular clones after completion of subsequent parallel sequencing. The use of the replicated substrate allows for rapid retrieval of the specific molecular clones in a non-contact mode.

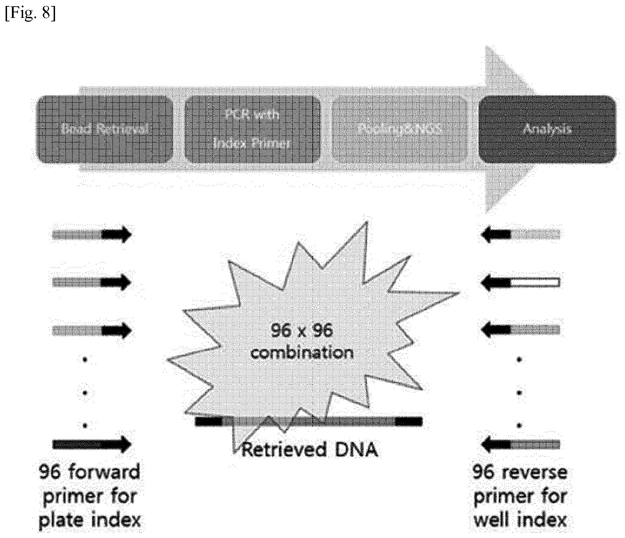

Fig. 8 is a schematic diagram illustrating a process for attaching DNA barcodes according to one embodiment of the present disclosure. The DNA barcodes are attached through combinations of forward primers and reverse primers. A combination of small numbers of primers (n for forward primers, m for reverse primers, a total of n+m) can give a large number of specific barcodes (n × m). Generally, the barcode at one side indicates the number of the well plate and the barcode at the other side indicates the number of the well on the well plate.

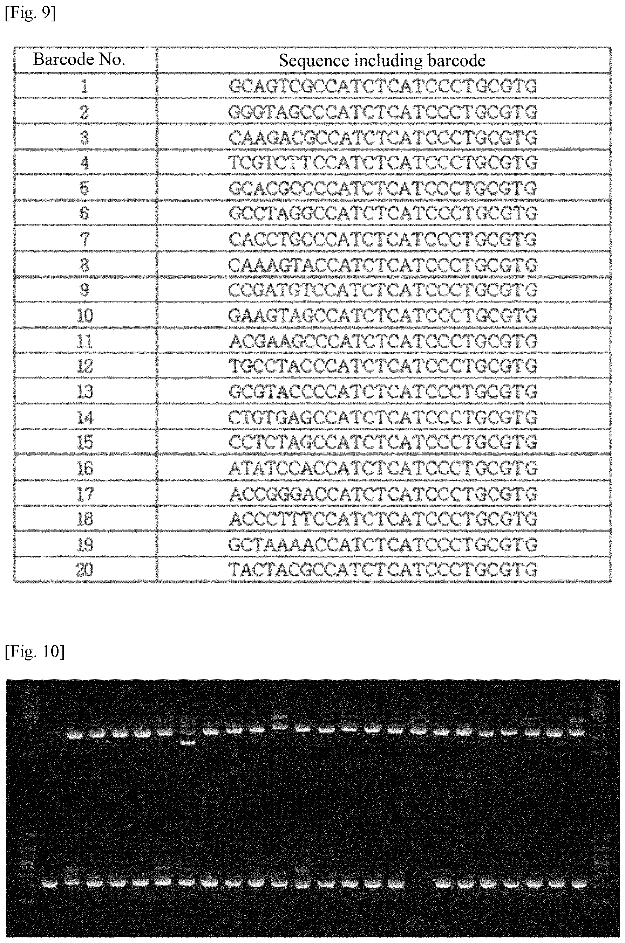

Fig. 9 shows the sequences of exemplary DNA barcodes used in experiments. Each barcode consists of 7 different bases corresponding to the barcode numbers and its sequence has no repeats of specific bases. The barcode sequence is generally located at the 5' end of the base sequence (the left side of the base sequence in Fig. 9). This facilitates the recognition of the barcode from the parallel sequencing results. However, the location of the barcode may vary can depending on the characteristics of a sequencer.

Fig. 10 shows amplification products of molecular clones attached with DNA barcodes according to exemplary embodiments of the present disclosure.

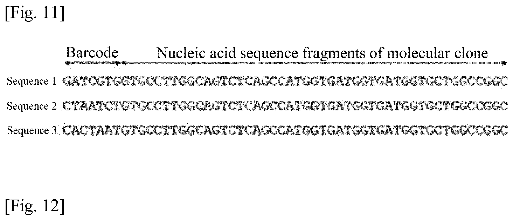

Fig. 11 shows some parallel sequencing results according to exemplary embodiments of the present disclosure. Each of the full-length sequences shown in Fig. 11 is divided into the sequence of the barcode and the nucleic acid sequence of the molecular clone. The location and sequence of the extracted molecular clone can be determined through sequencing and barcoding.

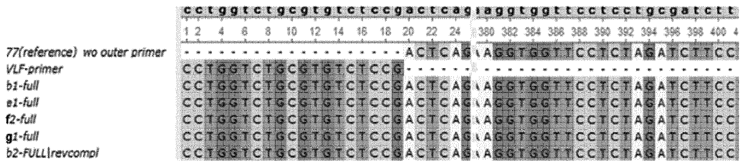

Fig. 12 shows the sequences of retrieved molecular clones according to exemplary embodiments of the present disclosure. The uppermost row (sample name: 77 (reference)) shows parallel sequencing results and the rest rows show the sequences of the retrieved molecular clones after cloning into *E. coli.* The sequences of the retrieved molecular clones were determined by Sanger sequencing. As can be seen from Fig. 12, the parallel sequencing results are the same as the actual sequences of the retrieved molecular clones, demonstrating that the present disclosure can be utilized for antibody sequencing or gene synthesis.

When the present disclosure is used for gene synthesis, the sequences of molecular clones can be analyzed at low cost, resulting in a significant reduction in the total cost of gene synthesis.

The present disclosure can also be utilized for target sequencing or the construction of capture probes for exome or whole exome sequencing (WES). For example, specific sequences can be selectively retrieved by characterizing desired molecular clones, selectively retrieving the desired molecular clones, and subjecting the retrieved molecular clones to amplification, transcription, and hybridization with gDNA. By analyzing the retrieved specific sequences, target sequencing or exome sequencing can be effectively performed.

The present disclosure can be applied to antibody synthesis and characterizing. For example, molecular clones are obtained from antibody-expressing microbes, bacteriophages or viruses, the antibody binding properties of the molecular clones with specific antigens are measured, desired ones of the molecular clones are extracted and sequenced, new antibodies with desired characteristics are synthesized from the desired molecular clones, and the antibodies are characterized.

As another example, molecular clones are obtained by extracting from antibody-expressing cells or bacteriophages, DNA barcodes are attached to the molecular clones and analyzed by NGS, molecular clones of interest are retrieved and inserted into pComb3x to produce antibodies or scFv proteins, the antibodies or proteins are characterized, and proteins with good binding properties and DNA sequences coding therefor are characterized.

In comparison with methods for extracting DNAs from sequencing substrates, the present disclosure possesses the following advantages.

First, methods for extracting DNAs from sequencing substrates have a limitation in selecting available next generation sequencing platforms. For example, DNAs can be easily extracted using a laser in a Roche 454 sequencer but there is a difficulty in applying extraction techniques to sequencing systems based on semiconductor chips using opaque substrates (for example, Ion Torrent Proton), general nanopore systems using fluidic channels or Illumina systems based on glass substrates. In contrast, the method of the present disclosure utilizes an NGS platform for sequencing only, avoiding the need to consider ease of DNA extraction. Therefore, the method of the present disclosure can be applied to all sequencing systems.

Second, DNAs extracted from sequencing substrates should be amplified for downstream applications. Generally, since the DNAs are very rare, the success rate of their amplification should be increased to around 100%. This involves considerable economic and time efforts, with the result that the cost of the entire procedure increases. In contrast, the method of the present disclosure can reduce the consumption of time and reagents to half or less while maintaining the success rate of amplification at 80% or more, resulting in a reduction in total cost.

Third, the method of the present disclosure can be potentially applied to any type of molecular clone because it is not affected by sequencing platforms. Particularly, the method of the present disclosure can use microbes that have been traditionally used in the field of molecular biology. For example, according to the present disclosure, microbes can be applied to study proteins (including antibodies) through surface display. The method of the present disclosure has at least 10 times higher accuracy during replication of molecular clones than *in vitro* amplification methods such as PCR.
## Claims

1. A method for extracting and characterizing molecular clones, comprising: providing a substrate on which molecular clones are formed; applying energy to desired ones of the molecular clones in a non-contact mode to extract the desired molecular clones from the substrate; chemically linking DNA barcodes to the sequences of the extracted molecular clones; and determining the DNA barcode-linked sequences by parallel sequencing.

2. The method according to claim 1, wherein the molecular clones are formed from a microbe.

3. The method according to claim 1, wherein the molecular clones are formed by emPCR.

4. The method according to claim 1, wherein the molecular clones take the form of microbial colonies with a diameter of 100 µm or less.

5. The method according to claim 1, wherein the substrate is selected from the group consisting of a substrate replicated from a template, a substrate comprising a sacrificial layer therein, a substrate surface coated with a sacrificial layer, and a substrate undergoing a phase transition under an electromagnetic field.

6. The method according to claim 1, wherein the substrate comprises a microwell structure as an array of regularly arranged wells, each of which has a volume of 1 µL or less.

7. The method according to claim 1, wherein the application of energy in a non-contact mode is performed by at least one mode selected from the group consisting of ultrasonic wave application, pneumatic pressure, and laser application, preferably wherein the application of energy in a non-contact mode is based on pulse laser ablation by an incident pulse laser or radiation pressure ejection.

8. The method according to claim 1, wherein the chemical linking is performed using an enzyme, preferably wherein the enzyme is a polymerase.

9. The method according to claim 8, wherein the barcodes are linked using the polymerase by multiplex PCR.

10. The method according to claim 1, wherein the DNA barcodes are chemically linked to both ends of the sequence of each extracted molecular clone.

11. The method according to claim 1, wherein the chemical linking is performed by combinatorial barcoding using two or more sets of DNA barcodes.

12. The method according to claim 1, further comprising retrieving only specific ones of the molecular clones analyzed by parallel sequencing.

13. The method according to claim 12, further comprising expressing and producing RNAs or proteins using the DNAs of the retrieved molecular clones.

14. The method according to claim 13, wherein the protein expression and production are accomplished using cells or bacteriophages.

15. The method according to claim 13, further comprising analyzing the structural features of the expressed proteins or the binding strengths of the expressed proteins to other substances.

## Patentansprüche

1. Verfahren zum Extrahieren und Charakterisieren molekularer Klone, umfassend: Bereitstellen eines Substrats, auf dem molekulare Klone gebildet werden; Anwenden von Energie auf gewünschte der molekularen Klone in einem kontaktlosen Modus, um die gewünschten molekularen Klone aus dem Substrat zu extrahieren; chemisches Verknüpfen von DNA-Strichcodes mit den Sequenzen der extrahierten molekularen Klone; und Bestimmen der DNA-Strichcode-verknüpften Sequenzen durch parallele Sequenzierung.

2. Verfahren nach Anspruch 1, bei dem die molekularen Klone aus einer Mikrobe gebildet werden.

3. Verfahren nach Anspruch 1, wobei die molekularen Klone durch emPCR gebildet werden.

4. Verfahren nach Anspruch 1, wobei die molekularen Klone die Form von Mikrobenkolonien mit einem Durchmesser von 100 µm oder weniger haben.

5. Verfahren nach Anspruch 1, wobei das Substrat aus der Gruppe ausgewählt wird, die aus einem aus einem Template replizierten Substrat, einem Substrat mit einer Opferschicht darin, einer mit einer Opferschicht beschichteten Substratoberfläche und einem Substrat, das unter einem elektromagnetischen Feld einen Phasenübergang durchläuft, besteht.

6. Verfahren nach Anspruch 1, wobei das Substrat eine Mikrovertiefungsstruktur als ein Array von regelmäßig angeordneten Vertiefungen umfasst, von denen jede ein Volumen von 1 µL oder weniger hat.

7. Verfahren nach Anspruch 1, wobei die Anwendung von Energie in einem kontaktlosen Modus durch mindestens einen Modus durchgeführt wird, der aus der Gruppe bestehend aus Ultraschallwellenanwendung, pneumatischem Druck und Laseranwendung ausgewählt wird, vorzugsweise wobei die Anwendung von Energie in einem kontaktlosen Modus auf Impulslaserablation durch einen einfallenden Impulslaser oder Strahlungsdruckausstoß basiert.

8. Verfahren nach Anspruch 1, wobei die chemische Verknüpfung unter Verwendung eines Enzyms durchgeführt wird, wobei das Enzym vorzugsweise eine Polymerase ist.

9. Verfahren nach Anspruch 8, bei dem die Strichcodes unter Verwendung der Polymerase durch Multiplex-PCR verknüpft werden.

10. Verfahren nach Anspruch 1, bei dem die DNA-Strichcodes chemisch mit beiden Enden der Sequenz jedes extrahierten molekularen Klons verbunden werden.

11. Verfahren nach Anspruch 1, bei dem die chemische Verknüpfung durch kombinatorische Strichcodierung unter Verwendung von zwei oder mehr Sätzen von DNA-Strichcodes durchgeführt wird.

12. Verfahren nach Anspruch 1, ferner umfassend die Gewinnung nur spezifischer der durch parallele Sequenzierung analysierten molekularen Klone.

13. Verfahren nach Anspruch 12, ferner umfassend die Expression und Herstellung von RNAs oder Proteinen unter Verwendung der DNAs der entnommenen molekularen Klone.

14. Verfahren nach Anspruch 13, bei dem die Proteinexpression und -herstellung unter Verwendung von Zellen oder Bakteriophagen erfolgt.

15. Verfahren nach Anspruch 13, ferner umfassend das Analysieren der strukturellen Merkmale der exprimierten Proteine oder der Bindungsstärken der exprimierten Proteine an andere Substanzen.

## Revendications

1. Procédé d'extraction et de caractérisation de clones moléculaires, comprenant : la fourniture d'un substrat sur lequel des clones moléculaires sont formés; l'application d'une énergie à ceux souhaités parmi les clones moléculaires dans un mode sans contact pour extraire les clones moléculaires souhaités à partir du substrat ; la liaison chimique de codes-barres à ADN aux séquences des clones moléculaires extraits ; et la détermination des séquences liées aux codes-barres à ADN par séquençage parallèle.

2. Procédé selon la revendication 1, dans lequel les clones moléculaires sont formés à partir d'un microbe.

3. Procédé selon la revendication 1, dans lequel les clones moléculaires sont formés par emPCR.

4. Procédé selon la revendication 1, dans lequel les clones moléculaires prennent la forme de colonies microbiennes d'un diamètre de 100 µm ou moins.

5. Procédé selon la revendication 1, dans lequel le substrat est sélectionné dans le groupe consistant en un substrat répliqué à partir d'un modèle, un substrat comprenant une couche sacrificielle à l'intérieur de celui-ci, une surface de substrat revêtue d'une couche sacrificielle, et un substrat subissant une transition de phase sous un champ électromagnétique.

6. Procédé selon la revendication 1, dans lequel le substrat comprend une structure à micropuits sous la forme d'un réseau de puits agencés régulièrement, dont chacun présente un volume de 1 µl ou moins.

7. Procédé selon la revendication 1, dans lequel l'application d'énergie dans un mode sans contact est effectuée par au moins un mode sélectionné dans le groupe consistant en l'application d'ondes ultrasonores, une pression pneumatique, et l'application d'un laser, de préférence dans lequel l'application d'énergie dans un mode sans contact est basée sur une ablation par laser pulsé par un laser pulsé incident ou une éjection de pression de rayonnement.

8. Procédé selon la revendication 1, dans lequel la liaison chimique est effectuée en utilisant une enzyme, de préférence dans lequel l'enzyme est une polymérase.

9. Procédé selon la revendication 8, dans lequel les codes-barres sont liés en utilisant la polymérase par PCR multiplex.

10. Procédé selon la revendication 1, dans lequel les codes-barres à ADN sont liés chimiquement aux deux extrémités de la séquence de chaque clone moléculaire extrait.

11. Procédé selon la revendication 1, dans lequel la liaison chimique est effectuée par codage à barres combinatoire en utilisant deux ensembles de codes-barres à ADN ou plus.

12. Procédé selon la revendication 1, comprenant en outre la récupération uniquement de certains spécifiques parmi les clones moléculaires analysés par séquençage parallèle.

13. Procédé selon la revendication 12, comprenant en outre l'expression et la production d'ARN ou de protéines en utilisant les ADN des clones moléculaires récupérés.

14. Procédé selon la revendication 13, dans lequel l'expression et la production de protéines sont accomplies en utilisant des cellules ou des bactériophages.

15. Procédé selon la revendication 13, comprenant en outre l'analyse des caractéristiques structurelles des protéines exprimées ou des forces de liaison des protéines exprimées pour d'autres substances.


<110> CELEMICS, INC.
<120> Isolation and identification method of molecular clone
<130> SDP2016-1053
<150> KR 10-2015-0154483
   <151> 2015-11-04
<150> KR 10-2016-0119420
   <151> 2016-09-19
<160> 30
<170> KoPatentIn 3.0
<210> 1
   <211> 25
   <212> DNA
   <213> Artificial Sequence
<220>
   <223> Barcode Number 1
<400> 1
   gcagtcgcca tctcatccct gcgtg 25
<210> 2
   <211> 25
   <212> DNA
   <213> Artificial Sequence
<220>
   <223> Barcode Number 2
<400> 2
   gggtagccca tctcatccct gcgtg 25
<210> 3
   <211> 25
   <212> DNA
   <213> Artificial Sequence
<220>
   <223> Barcode Number 3
<400> 3
   caagacgcca tctcatccct gcgtg 25
<210> 4
   <211> 25
   <212> DNA
   <213> Artificial Sequence
<220>
   <223> Barcode Number 4
<400> 4
   tcgtcttcca tctcatccct gcgtg 25
<210> 5
   <211> 25
   <212> DNA
   <213> Artificial Sequence
<220>
   <223> Barcode Number 5
<400> 5
   gcacgcccca tctcatccct gcgtg 25
<210> 6
   <211> 25
   <212> DNA
   <213> Artificial Sequence
<220>
   <223> Barcode Number 6
<400> 6
   gcctaggcca tctcatccct gcgtg 25
<210> 7
   <211> 25
   <212> DNA
   <213> Artificial Sequence
<220>
   <223> Barcode Number 7
<400> 7
   cacctgccca tctcatccct gcgtg 25
<210> 8
   <211> 25
   <212> DNA
   <213> Artificial Sequence
<220>
   <223> Barcode Number 8
<400> 8
   caaagtacca tctcatccct gcgtg 25
<210> 9
   <211> 25
   <212> DNA
   <213> Artificial Sequence
<220>
   <223> Barcode Number 9
<400> 9
   ccgatgtcca tctcatccct gcgtg 25
<210> 10
   <211> 25
   <212> DNA
   <213> Artificial Sequence
<220>
   <223> Barcode Number 10
<400> 10
   gaagtagcca tctcatccct gcgtg 25
<210> 11
   <211> 25
   <212> DNA
   <213> Artificial Sequence
<220>
   <223> Barcode Number 11
<400> 11
   acgaagccca tctcatccct gcgtg 25
<210> 12
   <211> 25
   <212> DNA
   <213> Artificial Sequence
<220>
   <223> Barcode Number 12
<400> 12
   tgcctaccca tctcatccct gcgtg 25
<210> 13
   <211> 25
   <212> DNA
   <213> Artificial Sequence
<220>
   <223> Barcode Number 13
<400> 13
   gcgtacccca tctcatccct gcgtg 25
<210> 14
   <211> 25
   <212> DNA
   <213> Artificial Sequence
<220>
   <223> Barcode Number 14
<400> 14
   ctgtgagcca tctcatccct gcgtg 25
<210> 15
   <211> 25
   <212> DNA
   <213> Artificial Sequence
<220>
   <223> Barcode Number 15
<400> 15
   cctctagcca tctcatccct gcgtg 25
<210> 16
   <211> 25
   <212> DNA
   <213> Artificial Sequence
<220>
   <223> Barcode Number 16
<400> 16
   gcagtcgcca tctcatccct gcgtg 25
<210> 17
   <211> 25
   <212> DNA
   <213> Artificial Sequence
<220>
   <223> Barcode Number 17
<400> 17
   accgggacca tctcatccct gcgtg 25
<210> 18
   <211> 25
   <212> DNA
   <213> Artificial Sequence
<220>
   <223> Barcode Number 18
<400> 18
   accctttcca tctcatccct gcgtg 25
<210> 19
   <211> 25
   <212> DNA
   <213> Artificial Sequence
<220>
   <223> Barcode Number 19
<400> 19
   gctaaaacca tctcatccct gcgtg 25
<210> 20
   <211> 25
   <212> DNA
   <213> Artificial Sequence
<220>
   <223> Barcode Number 20
<400> 20
   tactacgcca tctcatccct gcgtg 25
<210> 21
   <211> 54
   <212> DNA
   <213> Artificial Sequence
<220>
   <223> Sequence 1
<400> 21
   gatcgtggtg ccttggcagt ctcagccatg gtgatggtga tggtgctggc cggc 54
<210> 22
   <211> 54
   <212> DNA
   <213> Artificial Sequence
<220>
   <223> Sequence 2
<400> 22
   ctaatctgtg ccttggcagt ctcagccatg gtgatggtga tggtgctggc cggc 54
<210> 23
   <211> 54
   <212> DNA
   <213> Artificial Sequence
<220>
   <223> Sequence 3
<400> 23
   cactaatgtg ccttggcagt ctcagccatg gtgatggtga tggtgctggc cggc 54
<210> 24
   <211> 236
   <212> DNA
   <213> Artificial Sequence
<220>
   <223> 77(reference)_wo_outer_primer
<400> 24
   actcagggct ggtttcgcta ccgtggccca ggcggccgag ctcgtgatga cacagtctcc 60
   aggcaccctg tctttgtctc caggggagag agccaccctc tcctgcaggg ccagtcagag 120
   tgttagcagc agctacttag cctggtacca gcagaaacct ggccaggctc ccaggctcct 180
   catctatggt gcatccaaca gggccactgg catcccagac aggttcagtg gcagtg 236
<210> 25
   <211> 19
   <212> DNA
   <213> Artificial Sequence
<220>
   <223> VLF-primer
<400> 25
   cctggtctgc gtgtctccg 19
<210> 26
   <211> 877
   <212> DNA
   <213> Artificial Sequence
<220>
   <223> b1-full
<400> 26
   cctggtctgc gtgtctccga ctcagggctg gtttcgctac cgtggcccag gcggccgagc 60
   tcgtgatgac acagtctcca ggcaccctgt ctttgtctcc aggggagaga gccaccctct 120
   cctgcagggc cagtcagagt gttagcagca gctacttagc ctggtaccag cagaaacctg 180
   gccaggctcc caggctcctc atctatggtg catccaacag ggccactggc atcccagaca 240
   ggttcagtgg cagtgggtct gggacagact tcactctcac catcagcaga ctggagcctg 300
   aagattttgc agtgtattac tgtcagcagt atggtagctc accattcact ttcggccctg 360
   ggaccaaagt ggatatcaaa ggtggttcct ctagatcttc ctcctctggt ggcggtggct 420
   cgggcggggg tggggaggtg cagctggtgc agtctggggc tgaggtgaag aagcctgggt 480
   cctcggtgaa ggtctcctgc aaggcttctg gagacacctt cagcagctat gctatcagct 540
   gggtgcgaca ggcccctgga cacgggcttg agtggatggg aaggatcatc cctatctttg 600
   gtacaacaaa ctacgcacag aagttccagg gcagagtcac gactaccgcg gacgaatcca 660
   cgagcacagc ctacatggag ctgagcagcc tgagatctga ggacacggcc gtgtattact 720
   gtgcgagtag atccggagat tactatggtt cggggagtta ttcaaacttt gactactggg 780
   gccagggaac cctggtcacc gtctcctcag cctccaccaa gggcccatcg gtcactagtg 840
   gccaggccgg ccagcaccat caccatcacc ggccgac 877
<210> 27
   <211> 877
   <212> DNA
   <213> Artificial Sequence
<220>
   <223> e1-full
<400> 27
   cctggtctgc gtgtctccga ctcagggctg gtttcgctac cgtggcccag gcggccgagc      60
   tcgtgatgac acagtctcca ggcaccctgt ctttgtctcc aggggagaga gccaccctct     120
   cctgcagggc cagtcagagt gttagcagca gctacttagc ctggtaccag cagaaacctg     180
   gccaggctcc caggctcctc atctatggtg catccaacag ggccactggc atcccagaca     240
   ggttcagtgg cagtgggtct gggacagact tcactctcac catcagcaga ctggagcctg     300
   aagattttgc agtgtattac tgtcagcagt atggtagctc accattcact ttcggccctg     360
   ggaccaaagt ggatatcaaa ggtggttcct ctagatcttc ctcctctggt ggcggtggct     420
   cgggcggtgg tggggaggtg cagctggtgc agtctggggc tgaggtgaag aagcctgggt     480
   cctcggtgaa ggtctcctgc aaggcttctg gagacacctt cagcagctat gctatcagct     540
   gggtgcgaca ggcccctgga cacgggcttg agtggatggg aaggatcatc cctatctttg     600
   gtacaacaaa ctacgcacag aagttccagg gcagagtcac gactaccgcg gacgaatcca     660
   cgagcacagc ctacatggag ctgagcagcc tgagatctga ggacacggcc gtgtattact     720
   gtgcgagtag atccggagat tactatggtt cggggagtta ttcaaacttt gactactggg     780
   gccagggaac cctggtcacc gtctcctcag cctccaccaa gggcccatcg gtcactagtg     840
   gccaggccgg ccagcaccat caccatcacc ggccgac                              877
<210> 28
   <211> 877
   <212> DNA
   <213> Artificial Sequence
<220>
   <223> f2-full
<400> 28
   cctggtctgc gtgtctccga ctcagggctg gtttcgctac cgtggcccag gcggccgagc      60
   tcgtgatgac acagtctcca ggcaccctgt ctttgtctcc aggggagaga gccaccctct    120
   cctgcagggc cagtcagagt gttagcagca gctacttagc ctggtaccag cagaaacctg    180
   gccaggctcc caggctcctc atctatggtg catccaacag ggccactggc atcccagaca    240
   ggttcagtgg cagtgggtct gggacagact tcactctcac catcagcaga ctggagcctg    300
   aagattttgc agtgtattac tgtcagcagt atggtagctc accattcact ttcggccctg    360
   ggaccaaagt ggatatcaaa ggtggttcct ctagatcttc ctcctctggt ggcggtggct    420
   cgggcggtgg tggggaggtg cagctggtgc agtctggggc tgaggtgaag aagcctgggt    480
   cctcggtgaa ggtctcctgc aaggcttctg gagacacctt cagcagctat gctatcagct    540
   gggtgcgacg ggcccctgga cacgggcttg agtggatggg aaggatcatc cctatctttg    600
   gtacaacaaa ctacgcacag aagttccagg gcagagtcac gactaccgcg gacgaatcca    660
   cgagcacagc ctacatggag ctgagcagcc tgagatctga ggacacggcc gtgtattact    720
   gtgcgagtag atccggagat tactatggtt cggggagtta ttcaaacttt gactactggg    780
   gccagggaac cctggtcacc gtctcctcag cctccaccaa gggcccatcg gtcactagtg    840
   gccaggccgg ccagcaccat caccatcacc ggccgac                             877
<210> 29
   <211> 120
   <212> DNA
   <213> Artificial Sequence
<220>
   <223> g1-full
<400> 29
<210> 30
   <211> 877
   <212> DNA
   <213> Artificial Sequence
<220>
   <223> b2-FULL|revcompl
<400> 30
   cctggtctgc gtgtctccga ctcagggctg gtttcgctac cgtggcccag gcggccgagc      60
   tcgtgatgac acagtctcca ggcaccctgt ctttgtctcc aggggagaga gccaccctct     120
   cctgcagggc cagtcagagt gttagcagca gctacttagc ctggtaccag cagaaacctg     180
   gccaggctcc caggctcctc atctatggtg catccaacag ggccactggc atcccagaca     240
   ggttcagtgg cagtgggtct gggacagact tcactctcac catcagcaga ctggagcctg     300
   aagattttgc agtgtattac tgtcagcagt atggtagctc accattcact ttcggccctg     360
   ggaccaaagt ggatatcaaa ggtggttcct ctagatcttc ctcctctggt ggcggtggct     420
   cgggcggtgg tggggaggtg cagctggtgc agtctggggc tgaggtgaag aagcctgggt     480
   cctcggtgaa ggtctcctgc aaggcttctg gagacacctt cagcagctat gctatcagct     540
   gggtgcgaca ggcccctgga cacgggcttg agtggatggg aaggatcatc cctatctttg     600
   gtacaacaaa ctacgcacag aagttccagg gcagagtcac gactaccgcg gacgaatcca     660
   cgagcacagc ctacatggag ctgagcagcc tgagatctga ggacacggcc gtgtattact     720
   gtgcgagtag atccggagat tactatggtt cggggagtta ttcaaacttt gactactggg     780
   gccagggaac cctggtcacc gtctcctcag cctccaccaa gggcccatcg gtcactagtg     840
   gccaggccgg ccagcaccat caccatcacc ggccgac                              877